(19) Europäisches Patentamt
European Patent Office
Office européen des brevets

(11) EP 1 924 159 B1

(12) # EUROPEAN PATENT SPECIFICATION

(45) Date of publication and mention of the grant of the patent: **19.10.2011 Bulletin 2011/42**

(21) Application number: **06783327.7**

(22) Date of filing: **18.07.2006**

(51) Int Cl.:
***A61K 36/185*** *(2006.01)* ***A23L 1/29*** *(2006.01)*
***A61P 37/08*** *(2006.01)*

(86) International application number:
**PCT/KR2006/002807**

(87) International publication number:
**WO 2007/011148 (25.01.2007 Gazette 2007/04)**

(54) **A composition comprising an extract of Tiarella polyphylla having antiasthmatic activity**

Zusammensetzung enthaltend einen Extrakt von Tiarella polyphylla mit antiasthmatischer Wirkung

Composition comprenant un extrait de Tiarella polyphylla présentant une activité antiasthmatique

(84) Designated Contracting States:
**AT BE BG CH CY CZ DE DK EE ES FI FR GB GR HU IE IS IT LI LT LU LV MC NL PL PT RO SE SI SK TR**

(30) Priority:
**18.07.2005 KR 20050064669**
**18.07.2005 KR 20050064668**
**18.07.2006 KR 20060066861**
**18.07.2006 KR 20060066866**

(43) Date of publication of application:
**28.05.2008 Bulletin 2008/22**

(73) Proprietor: **Korea Research Institute of Bioscience and Biotechnology**
**Daejeon 305-806 (KR)**

(72) Inventors:
- **LEE, Hyeong Kyu**
**Yuseong-gu, Daejeon 305-755 (KR)**
- **OH, Sei Ryang**
**Seou-gu, Daejeon 302-243 (KR)**
- **AHN, Kyung Seop**
**Yuseong-su, Daejeon 305-325 (KR)**
- **LEE, Joong Ku**
**Yuseong-gu, Daejeon 305-761 (KR)**
- **LEE, San Ku**
**Yuseong-gu, Daejeon 305-330 (KR)**
- **KIM, Jung Hee**
**Yuseong-gu, Daejeon 305-752 (KR)**
- **KIM, Doo Young**
**Yuseong-gu, Daejeon 305-333 (KR)**
- **QUAN, Gui Hua**
**Natural Medicines Research Center**
**52, Eocun-dong**
**Yusoeng-gu**
**Daejeon 305-806 (KR)**
- **KWON, Ok Kyoung**
**Yuseong-gu, Daejeon 305-503 (KR)**
- **KIM, Eun Ah**
**Naju-si, Jeollanam-do 520-824 (KR)**
- **CHOI, Soon Ja**
**Yuseong-gu, Daejeon 305-752 (KR)**
- **SHEN, Guang Hai**
**Yuseong-gu, Daejeon 305-806 (KR)**
- **KIM, Mi Jin**
**Yuseong-gu, Daejeon 305-345 (KR)**
- **PARK, Bo Young**
**Yuseong-gu, Daejeon 305-390 (KR)**
- **MIN, Byung Sun**
**Seo-gu, Daejeon 302-120 (KR)**
- **HAN, Gyoon Hee**
**209-1702 Sinyeongtong Hyundai Apt.**
**Hwaseong-si, Gyeonggi-do 445-973 (KR)**
- **PARK, Hea Young**
**Gangnam-gu, Seoul 135-110 (KR)**
- **KIM, Gun Cheol**
**Yuseong-gu, Daejeon 305-390 (KR)**
- **KIM, Joo Heon**
**Yuseong-gu, Daejeon 305-345 (KR)**
- **KIM, So Young**
**Dong-gu, Daejeon 300-020 (KR)**

(74) Representative: **Behnisch, Werner**
**Reinhard, Skuhra, Weise & Partner GbR**
**Patent- und Rechtsanwälte**
**Friedrichstrasse 31**
**80801 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 924 159 B1

(56) References cited:
**EP-A2- 1 213 027** **WO-A1-02/102396**
**JP-A- 04 346 911** **KR-A- 20030 042 123**

- **DATABASE WPI 28 May 2003 (2003-05-28) Thomson Scientific, London, GB; AN 2003-826125 XP002599274 "Triterpene compound having apoptosis induction effect" & KR 2003 042 123 A ((KRIB ) KOREA RES INST BIOSCIENCE & BIOTECHNOLOG) 28 May 2003 (2003-05-28) -& KR 2003 0042123 A (KOREA RES INST OF BIOSCIENCE [KR]) 28 May 2003 (2003-05-28)**
- **PARK S H ET AL: "STRUCTURE DETERMINATION OF A NEW LUPANE-TYPE TRITERPENE, TIARELLIC ACID, ISOLATED FROM TIARELLA POLYPHYLLA" ARCHIVES OF PHARMACAL RESEARCH, NATL. FISHERIES UNIVERSITY, PUSAN, KR, vol. 25, no. 1, 1 January 2002 (2002-01-01), pages 57-60, XP008076470 ISSN: 0253-6269**
- **PARK S.H. ET AL.: 'Anticomplement activities of oleanolic acid monodesmosides an bisdesmosides isolated from Tiarella polyphylla' ARCH. PHARM. RES. vol. 22, no. 4, 1999, pages 428 - 431, XP008075591**
- **MOON H.I. ET AL.: 'Triterpenoid from Tiarella polyphylla, regulation of type 1 procollagen and MMP-1 in ultraviolet irradiation of cultured old age human dermal fibroblasts' ARCH. PHARM. RES. vol. 27, no. 10, 2004, pages 1060 - 1064, XP008075590**
- **PARK S.H. ET AL.: 'Structure determination of a new lupane-type triterpene, tiarellic acid, isolated from Tiarella polyphylla' ARCH. PHARM. RES. vol. 25, no. 1, 2002, pages 57 - 60, XP008076470**

## Description

## Technical Field

The present invention relates to a composition comprising a butanol soluble fraction of Tiarella polyphylla methanol extract having anti-asthmatic activity.

## Background Art

Asthma has been regarded as a complex syndrome occurring in the airways, which shows various disorders such as airflow obstruction, acute or chronic inflammation, airway hyper-responsiveness (AHR) and structural remodeling (Kumar R. K. Pharmacol. Ther., 91, pp 93-104, 2001).

Allergic inflammation occurring in the airways has been reported to play a critical role in asthma development and the number of patients suffering from allergic asthma has been increased to about 10% of the population in the world recently. It has been reported that the number has been reached to seventeen million in America and the market scale of the medication for allergic asthma has been enlarged to 640 billion $ in America till now.

Asthma can be classified into two types, i.e., extrinsic asthma and intrinsic asthma. Extrinsic asthma caused by the exposure of antigen such as house dust mite Dermatophagoides as a main antigen, pollen, epithelium of animal, fungi etc shows positive reaction in skin test or bronchial provocation test against the antigen, and generally occurs in younger people. Intrinsic asthma caused by upper respiratory infection, exercise, emotional instability, cold weather, the change of humidity occurs in adult patients.

According to the aspect of pathophysiology, asthma has been recognized as a chronic inflammation occurred by following procedure; Inflammatory cells are proliferated, differentiated, and activated caused by cytokines reproducing in T-helper 2 immune cells and is moved to air way or neighboring tissue thereof. The activated inflammatory cells such as neutrophil, mast cell etc release a variety of inflammatory mediators, such as cytokines, chemokines, signaling molecules, adhesion molecules and growth factors and the structural cells in airways are involved in various stages of asthma (Elias JA et al., J Clin Invest., 111, pp 291-7, 2003). In numerous studies using knockout mice models and clinical research, the critical observations in asthma could fall into several characteristic parameters, such as immune responses, eosinophilia, AHR and structural remodeling (Moffatt JD. Pharmacol Ther, 107, pp 343-57, 2005; Spina D et al., Trends Pharmacol Sci, 23, pp 311-5, 2002). Each of the parameters seems not to have direct correlations with one another; however, IgE-mediated immune response and eosinophilia are prominent symptoms in the airways of allergic asthma (Bochner B.S. et al., Annu. Rev. Immunol., 12, pp 295-335, 1994; Bousquet J et al., N. Engl. J. Med., 323, pp 1033-9, 1990), and the produced cytokines such as IL-4, IL-5 and IL-13 in the allergic process also play an important role in AHR development and airway remodeling (Riffo-Vasquez Y et al., Pharmacol. Ther., 94, pp 185-211, 2002). Indeed, asthma is a result of orchestrated inflammatory events, many of which involve specific inhibitors acting on the pathway of asthma, for example, histamine H1 antagonists, thromboxane antagonists, platelet-activating-factor antagonists, cyclooxygenase inhibitors, nitrogen monooxygenase inhibitors and prostaglandin inhibitors, have been tried but have failed in clinical trials (Moffatt J.D., Pharmacol. Ther., 107, pp 343-57, 2005). In contrast, glucocorticoids, which suppress the progenitor levels of inflammatory cells to baseline by widespread inhibition of cytokine synthesis and cytokine mediated immune-cell survival, has been used to manage the symptoms of asthmatic patients over a period of 30 years as far (Baatjes A.J. et al., Pharmacol, Ther., 95, pp 63-72, 2002). These reports suggest that the therapeutic approach for asthma management should focus on restoring the balance of asthmatic parameters rather than searching for potent inhibitors of specific pathways of the asthmatic process.

*Tiarella polyphylla* D.Don (Saxifragaceae) is a single species belonged to the genus in Korea. It inhabits in South-West in China but only on the summit of Ullung Island in Korea. Previously, it has been isolated tiarellic acid with corosolic acid, tormentic acid and so on (Park et al., Arch Pharm Res., 25, pp 57-60, 2002), and others reported an inhibitory effect on the expression of MMP-1 and type 1 procollagen in UV-irradiated skin fibroblasts(Moon et al., J Ethnopharmacol., 98, pp 185-189, 2005).

However, there has been not reported or disclosed about the suppressive effect on inflammatory, allergic and asthmatic disease of the extract of *Tiarella polyphylla* and tiarellic acid isolated therefrom in any of above cited literatures.

Accordingly, the present inventors have discovered that the butanol soluble fraction of Tiarella polyphylla methanol extract and tiarellic acid isolated therefrom show the inhibitory effect of tiarellic acid against $LTC_4$ release in vitro and the suppressive effect on the IgE level and the cytokine(IL-4, IL-5 and IL-13) production, airway hyperresponsiveness, and leukocyte infiltration in OVA-induced asthmatic mice for the expectiation of contribution to asthma management.

## Disclosure of Invention

### Technical Problem

Accordingly, there have been still needed to discover more effective drug to treat and prevent inflammatory, allergic and asthmatic disease without toxicity till now.

### Technical Solution

Accordingly, it is an object of the present invention to provide a composition comprising a crude extract or organic solvent soluble extract of *T. polyphylla, as* an active ingredient for the treatment and prevention of asthmatic disease.

The term "crude extract" disclosed herein comprises the extract prepared by extracting plant material with methanol.

The term "organic solvent soluble extract" disclosed herein is prepared by extracting the above described crude extract with butanol.

Tiarellic acid can be isolated from a crude extract or organic solvent soluble extract of *T. polyphylla,* represented by following chemical formula (I)

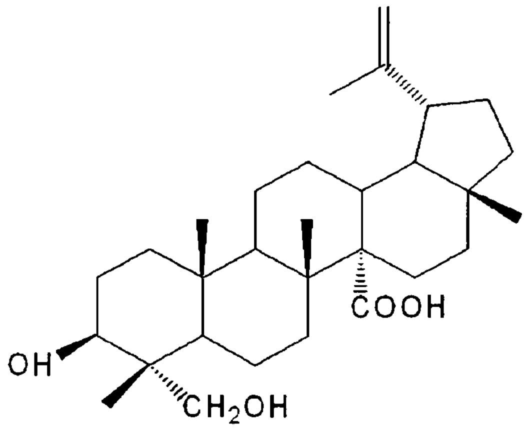

The present invention provides a use of crude extract or organic solvent soluble extract of *T. polyphylla* for manufacture of medicines employed for treating or preventing asthmatic disease.

In accordance with another aspect of the present invention, there is also provided a method of treating or preventing inflammatory, allergic and asthmatic disease in mammals, wherein the method comprises administering a therapeutically effective amount of crude extract or organic solvent soluble extract of *T. polyphylla* acidisolated therefrom into the mammal suffering with inflammatory, allergic and asthmatic disease.

An inventive extract isolated from *T. polyphylla* is be prepared in accordance with the following preferred embodiment.

Hereinafter, the present invention is described in detail.

For the present invention, the dried whole plant of *T. polyphylla* is cut into small pieces and the piece was mixed with methanol, and was heated at the temperature ranging from 20 to 100°C, preferably from 20 to 50°C, for the period ranging 10 to 48 hours, preferably 20 to 30 hours, by reflux extraction with hot water, cold water extraction, ultra-sonication or conventional extraction, preferably by cold water extraction; the residue was filtered and then the filtrate is dried to obtain polar solvent soluble extract thereof.

In the above crude extract prepared by above step, is suspended in water, and then is mixed with 1 to 100-fold, preferably, 1 to 5-fold volume of organic solvent butanol, to obtain organic solvent soluble extract of the present invention.

The above organic solvent soluble extract is further subjected to silica gel column chromatography(70-230 mesh, 8.5 x 65cm) filled with silicagel eluting with mixture solvent of n-hexane:ethyl acetate(ethyl acetate 10-20%, step gradient), and a chloroform:methanol(methanol 0-100%, step gradient) to obtain 9 fractions. Among the fractions, the 6 fraction is further subjected to repeated silica gel column chromatography using a normal phase silica column (silica gel, 230-400 mesh, 6.0 x 60cm, chloroform-methanol mixture, methanol 5-50% step gradient) to obtain the tiarellic acidof the present invention. The structure was confirmed by NMR($^1$H, $^{13}$C, DEPT, HMQC, HMBC), EI-MS and optical rotation with those reported previously(Park et al., Arch Pharm Res., 25, pp 57-60, 2002), and the purity was analyzed as more

than 99.5% by HPLC system(Shimadzu SCL-10A woth SPD-M 10A vp PDA detector, column; phenomenex Synergi 4 um Fusion RP-80, 4.6 x 150nm, elution: ACN/0.1 % TFA in DW, 4/1, v/v).

In accordance with another aspect of the present invention, there is provided a pharmaceutical composition comprising a crude extract and organic solvent soluble extract of *T. polyphylla* prepared by the above described preparation method for use in the treatment and prevention of asthmatic disease as active ingredients.

In accordance with another aspect of the present invention, there is also provided a use of a crude extract and organic solvent soluble extract of *T. polyphylla* prepared by the above described preparation method for manufacture of medicines employed for treating or preventing asthmatic disease.

In accordance with another aspect of the present invention, a crude extract and organic solvent soluble extract of *T. polyphylla* prepared by the above describe preparation method, is used for treating or preventing inflammatory, allergic and asthmatic disease, wherein the method comprises administering a therapeutically effective amount of comprising.

Compound represented by chemical formula (I) can be transformed into their pharmaceutically acceptable salt and solvates by the conventional method well known in the art. For the salts, acid-addition salt thereof formed by a pharmaceutically acceptable free acid thereof is useful and can be prepared by the conventional method. For example, after dissolving the compound in the excess amount of acid solution, the salts are precipitated by the water-miscible organic solvent such as methanol, ethanol, acetone or acetonitrile to prepare acid addition salt thereof and further the mixture of equivalent amount of compound and diluted acid with water or alcohol such as glycol monomethylether, can be heated and subsequently dried by evaporation or filtrated under reduced pressure to obtain dried salt form thereof.

As a free acid of above-described method, organic acid or inorganic acid can be used. For example, organic acid such as methansulfonic acid, p-toluensulfonic acid, acetic acid, trifluoroacetic acid, citric acid, maleic acid, succinic acid, oxalic acid, benzoic acid, lactic acid, glycolic acid, gluconic acid, galacturonic acid, glutamic acid, glutaric acid, glucuronic acid, aspartic acid, ascorbic acid, carbonylic acid, vanillic acid, hydroiodic acid and the like, and inorganic acid such as hydrochloric acid, phosphoric acid, sulfuric acid, nitric acid, tartaric acid and the like can be used herein.

Further, the pharmaceutically acceptable metal salt form may be prepared by using base. The alkali metal or alkali-earth metal salt thereof can be prepared by the conventional method, for example, after dissolving the compound in the excess amount of alkali metal hydroxide or alkali-earth metal hydroxide solution, the insoluble salts are filtered and remaining filtrate is subjected to evaporation and drying to obtain the metal salt thereof. As a metal salt, sodium, potassium or calcium salt are pharmaceutically suitable and the corresponding silver salt can be prepared by reacting alkali metal salt or alkali-earth metal salt with suitable silver salt such as silver nitrate.

The pharmaceutically acceptable salt of the present compound comprise all the acidic or basic salt which may be present at the compounds, if it does not indicated specifically herein. For example, the pharmaceutically acceptable salt comprise the salt of hydroxyl group such as the sodium, calcium and potassium salt thereof; the salt of amino group such as the hydrogen bromide salt, sulfuric acid salt, hydrogen sulfuric acid salt, phosphate salt, hydrogen phosphate salt, dihydrophosphate salt, acetate salt, succinate salt, citrate salt, tartarate salt, lactate salt, mandelate salt, methanesulfonate(mesylate) salt and p-toluenesulfonate (tosylate) salt etc, which can be prepared by the conventional method well known in the art.

The inventive composition for treating and preventing asthmatic disease may comprises the above described extracts or compounds as 0.1~ 50% by weight based on the total weight of the composition.

The composition according to the present invention can be provided as a pharmaceutical composition containing pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a patient by employing any of the procedures well known in the art.

For example, the compositions of the present invention can be dissolved in oils, propylene glycol or other solvents that are commonly used to produce an injection. Suitable examples of the carriers include physiological saline, polyethylene glycol, ethanol, vegetable oils, isopropyl myristate, etc., but are not limited to them. For topical administration, the extract of the present invention can be formulated in the form of ointments and creams.

Pharmaceutical formulations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

The composition of the present invention in pharmaceutical dosage forms may be used in the form of their pharmaceutically acceptable salts, and also may be used alone or in appropriate association, as well as in combination with other pharmaceutically active compounds.

The desirable dose of the inventive extract or compound varies depending on the condition and the weight of the subject, severity, drug form, route and period of administration, and may be chosen by those skilled in the art. However, in order to obtain desirable effects, it is generally recommended to administer at the amount ranging from 0.0001 to 100mg/kg, preferably, 0.001 to 10mg/kg by weight/day of the inventive extract of the present invention. The dose may be administered in single or divided into several times per day.

The pharmaceutical composition of present invention can be administered to a subject animal such as mammals (rat, mouse, domestic animals or human) via various routes. All modes of administration are contemplated, for example, administration can be made orally, rectally or by intravenous, intramuscular, subcutaneous, intra-cutaneous, intrathecal, epidural or intracerebroventricular injection.

It is the other object of the present invention to provide a functional health food comprising the extract or compounds isolated from *T. polyphylla* together with a sitologically acceptable additive for the prevention and alleviation of inflammatory, allergic and asthmatic disease.

To develop for functional health food, examples of addable food comprising the above extracts or compounds of the present invention are various food, beverage, gum. vitamin complex, health improving food and the like, and can be used as powder, granule, tablet, chewing tablet, capsule or beverage etc.

The above described composition therein can be added to food, additive or beverage, wherein, the amount of the above described extract or compound in food or beverage may generally range from about 0.01 to 80w/w%, preferably 0.01 to 15w/w% of total weight of food for the health food composition and 0.02 to 5g, preferably 0.3 to 1 g on the ratio of 100ml of the health beverage composition.

Providing that the health beverage composition of present invention contains the above described extract or compound as an essential component in the indicated ratio, there is no particular limitation on the other liquid component, wherein the other component can be various deodorant or natural carbohydrate etc such as conventional beverage. Examples of aforementioned natural carbohydrate are monosaccharide such as glucose, fructose etc; disaccharide such as maltose, sucrose etc; conventional sugar such as dextrin, cyclodextrin; and sugar alcohol such as xylitol, and erythritol etc. As the other deodorant than aforementioned ones, natural deodorant such as taumatin, stevia extract such as levaudioside A, glycyrrhizin et al., and synthetic deodorant such as saccharin, aspartam et al., may be useful favorably. The amount of above described natural carbohydrate is generally ranges from about 1 to 20 g, preferably 5 to 12 g in the ratio of 100 □ of present beverage composition.

The other components than aforementioned composition are various nutrients, a vitamin, a mineral or an electrolyte, synthetic flavoring agent, a coloring agent and improving agent in case of cheese chocolate et al., pectic acid and the salt thereof, alginic acid and the salt thereof, organic acid, protective colloidal adhesive, pH controlling agent, stabilizer, a preservative, glycerin, alcohol, carbonizing agent used in carbonate beverage et al. The other component than aforementioned ones may be fruit juice for preparing natural fruit juice, fruit juice beverage and vegetable beverage, wherein the component can be used independently or in combination. The ratio of the components is not so important but is generally range from about 0 to 20 w/w % per 100 w/w % present composition. Examples of addable food comprising aforementioned extract therein are various food, beverage, gum, vitamin complex, health improving food and the like.

Inventive extract of the present invention has no toxicity and adverse effect therefore they can be used with safe.

The present invention is more specifically explained by the following examples. However, it should be understood that the present invention is not limited to these examples in any manner.

**Advantageous Effects**

The present invention provides a pharmaceutical composition and a health food comprising an extract of *T. polyphylla* as an active ingredient in an effective amount to treat and prevent asthmatic disease.

**Brief Description of the Drawings**

The above and other objects, features and other advantages of the present invention will more clearly understood from the following detailed description taken in conjunction with the accompanying drawings, in which;

Fig. 1 shows the effects of an extract of *T. polyphylla* and tiarellic acid on lung tissue cell using by the histological examination of bronchoalveolar lavage (A: normal control mice, B: PBS-treated mice, C: *T. polyphylla* extract-treated mice, D: tiarellic acid-treated mice),

Fig. 2 shows the inhibitory effects of an extract of *T. polyphylla* and tiarellic acid on OVA-induced inflammation in lung tissue.

## Best Mode for Carrying Out the Invention

The present invention is more specifically explained by the following examples. However, it should be understood that the present invention is not limited to these examples in any manner.

## Mode for the Invention

The present invention is more specifically explained by the following examples. However, it should be understood that the present invention is not limited to these examples in any manner.

The following Reference Example, Examples and Experimental Examples are intended to further illustrate the present invention without limiting its scope.

**Example 1. Preparation of the crude extract of *T. polyphylla***

*T. polyphylla* was collected in August 2003, Ullung Island, Korea and the voucher specimen (PEB 3068) was deposited in the Plant Extract Bank of Korea Research Institute of Bioscience and Biotechnology, KRIBB) located in Daejeon, Korea.

1.1kg of dried *T. polyphylla* was cut into small pieces, mixed with 5L of methanol and the mixture was stirred at room temperature for 24 hours, extracted with cold water at three times. The extract was filtered with filter paper to remove the debris. The filtrate was pooled and concentrated by rotary evaporator 55~65°C under reduced pressure and dried with freezing dryer to obtain 100.5g of dried crude extract of *T. polyphylla.*

**Example 2. Preparation of butanol soluble fraction**

1 L of distilled water was added to 100.5 g of the crude extract obtained in Example 1. 1L of butanol was added thereto in separatory funnel and shaken vigorously to divide into butanol soluble layer and water soluble layer.

Above butanol soluble layer was concentrated with rotary evaporator, dried with freeze dryer to obtain butanol soluble extract, and finally, 80.0g of butanol soluble extract and water soluble extract were obtained, to use as a sample in the following experiments.

**Example 3. Preparation of tiarellic acid from the extract of *T. polyphylla***

3.29kg of the dried whole plants of *T. polyphylla* extracted with methanol(10L) twice at room temperature to obtain 352g of an extract. This extract was suspended in 1L of water and partitioned with an equal volume of n-hexane. 65.1g of the n-hexane soluble fraction was then subjected to a silica gel column chromatography (70-230 mesh, 8.5×65cm) and eluted with a n-hexane-ethyl acetate mixture (ethyl acetate 10-20%, step gradient), and a chloroform-methanol mixture (methanol 0-100%, step gradient), successively, and yielded 9 fractions (Fr. 1-Fr.9). 7.4g of the Fr. 6 (between chloroform-methanol 9/1-7/3, v/v) was subjected to column chromatography using a normal phase silica column (silica gel, 230-400 mesh, 6.0×60 cm, chloroform-methanol mixture, methanol 5-50% step gradient) to yield 400mg of tiarellic acid. The structure was confirmed by NMR ($^1H$, $^{13}C$, DEPT, HMQC, HMBC), EI-MS and optical rotation with those reported previously (Park et al., Arch Pharm Res., 25, pp 57-60, 2002) and the purity more than 99.5% was analyzed by HPLC system (Shimadzu SCL-10A with SPD-M 10A vp PDA detector, column; Phenomenex Synergi 4 um Fusion RP-80, 4.6×50 mm, elution: ACN/0.1% TFA in DW, 4/1, v/v).

<u>Tiarellic acid</u>

Needles (MeOH);

mp 254-256°C ;

[a] 2 3 D +94 (pyridine, c 0.14);

IR (KBr, $cm^{-1}$): 3491 (OH), 1689 (CO), 1645 (C=C), 1450, 1388, 1262, 1222, 1044; HRMS m/z 472.3552 ($M^+$, Calcd for $C_{30}H_{48}O_4$: 472.3553);

EIMS (rel. int.) *m/z*: 472 $[M]^+$ (61), 454 $[M-H_2O]^+$ (34), 436 $[M-2H_2O]^+$ (62), 424 (42), 396 (26), 205 (75), 187 (71), 175 (87), 173 (100);

$^{13}C$-NMR (150 MHz, pyridine-$d_5$): 13.0 (C-24), 17.4 (C-25), 17.5 (C-26), 18.7 (C-6), 18.8 (C-28), 19.4 (C-30), 21.3 (C-11), 25.8 (C-15), 26.7 (C-12), 27.9 (C-2), 30.1 (C-21), 37.7 (C-10), 38.2 (C-7), 38.3 (C-16), 39.2 (C-1), 39.6 (C-13), 40.4 (C-22), 40.8 (C-8), 42.9 (C-4), 43.0 (C-17), 48.1 (C-19), 49.2 (C-5), 51.4 (C-18), 51.6 (C-9), 60.4 (C-14), 68.2 (C-23), 73.6 (C-3), 110.2 (C-29), 150.9 (C-20), 178.3 (C-27).

$^1H$-NMR (600 MHz, pyridine-*d*5): 1.05, 1.71 (2H, m, each, H-1), 1.82, 1.91 (2H, m, each, H-2), 4.02 (1H, dd, *J* = 4.7, 11.6 Hz, H-3), 1.51 (1H, dd, *J* = 1.5, 12.0 Hz, H-5), 1.48, 1.65 (2H, m, each, H-6), 1.87, 2.06 (2H, m, each, H-7), 2.02 (1H, dd, *J* = 1.7, 12.7 Hz, H-9), 1.32, 1.64 (2H, m, each, H-11), 1.87, 2.60 (2H, m, each, H-12), 1.88 (1H, m, H-13), 1.67, 2.28 (2H, m, each, H-15), 1.70, 1.78 (2H, m, each, H-16), 1.81 (1H, m, H-18), 2.60 (1H, m, H-19), 1.36, 1.97 (2H, m, each, H-21), 1.16, 1.40 (2H, m, each, H-22), 3.57, 4.07 (2H, d, *J* = 10.4, each, H-23), 1.04 (3H, s, H-24), 1.01 (3H, s, H-25), 1.21 (3H, s, H-26), 0.90 (1H, s, H-28). 4.76, 4.96 (2H, s, each, H-29), 1.86 (3H, d, *J* = 6.4 Hz, H-30),

**Experimental Example 1. Animal sensitization and airway challenge**

Groups of mice (n=5-6) were studied; they received the following treatment: (1) sham-sensitization plus challenge with phosphate-buffered saline (PBS; ipNeb); (2) sensitization plus challenge with OVA (ovalbumin: Sigma A5503;

Sigma, St. Louis, MO) (ipNeb); (3) sensitization with OVA (ip) plus challenge with OVA (Neb) and samples (tiarellic acid or zileuton, po). Briefly, mice were sensitized with intraperitoneal injection of 20μg OVA, which was emulsified with 2 mg of aluminum hydroxide in 100μl of PBS buffer (pH 7.4) on days 0 and 11. The mice were challenged through the airways with OVA (1% in PBS) for 20 min using an ultrasonic nubuilizer (NE-U12; Omron Corp., Tokyo, Japan) on days 19, 20, 21 and 25 after the initial sensitization. The mice were sacrificed 48 hours after the last challenge (day 27) to determine the suppressive effect of extract of *T. polyphylla* and tiarellic acid on the airways of allergic asthma.

**Experimental Example 2. MTT assay**

To investigate the cytotoxic activity of inventive extract of *T. polyphylla* and tiarellic acid isolated therefrom, (3-[4,5-dimethylthiazol-2-yl]-2,5-diphenyl tetrazolium bromide(MTT) assay method was performed by following procedure (Wang Z et al., Biol., Pharm. Bull., 24, pp 159-162, 2001).

Promyelotic HL-60 cells (HL-18103, $5\times10^5$ cells/ml) were seeded on 96-well plates under NGF-free condition. After 24 hours incubation, the cells were treated with the mixture of samples dissolved in 10μl of DMSO and 10μl of MTT solution (5mg/ml), and incubated for 4 hours under the similar condition. 4 hours later, MTT was removed and 100μl of DMSO was dropped into each well to dissolve crystals. UV absorbance was measured by microplate reader (BIO-RAD, U.S.A.) at 570nm to calculate the cell viability.

As shown in Table 1, it is confirmed that an inventive extract or compound the present invention did not show cell toxicity.

Table 1

| Effect of extract and compound isolated from *T. polyphylla* on HL-60 cells. | | |
|---|---|---|
| Sample | Cell viability (%) | |
| | 50μM | 100μM |
| *T. polyphylla* extract | 100 | 102 |
| Tiarellic acid | 100 | 102 |

**Experimental Example 3. Preparation and Activation of Bone Marrow-Derived Mast Cells(BMMC)**

BMMC were obtained from male Balb/c mice and cultured for up to 4 weeks in 50% enriched medium (RPMI containing 2 mM L-glutamine, 25 mM HEPES buffer, 2 mg/ml sodium bicarbonate, 100 units/ml penicillin G, 100μg/ml streptomycin sulfate, 0.25μg/ml amphotericin B) supplemented with 10% fetal bovine serum with IL-3 (Sigma I4144, 2 ng/ml)(Murakami M et al., J. Bio. Chem., 39, pp 22653-22656, 1995). 3 weeks after the culture, more than 98% of BMMC was found in the cells as assessed by staining method with toluidine blue.

BMMC were suspended in the enriched medium at the cell density of $1\times10^6$ cells/ml, and were then incubated in a humidified 5% $CO_2$ incubator with or without sample in DMSO (final DMSO concentration was less than 0.5%) for 30 min at 37°C. After the stimulation with stem cell factor (SCF, Sigma S9915, 100ng/ml) for 20 min, the $LTC_4$ release in supernatants was measured by an enzyme immunoassay kit (Cayman Chemical, Ann Arbor, MI, U.S.A.) according to the manufacturer's instructions. All the experiments were carried out in triplicate and the inhibition of $LTC_4$ release was determined by calculating % reduction of $LTC_4$ release from the control(Lee SH et al., Biol. Pharm. Bull., 27, pp786-788, 2004).

**Experimental Example 4. The effect of the extract of *T. polyphylla* and tiarellic acid on $LTC_4$ release**

As described in Experimental Example 3, the inhibitory effect of the extract of *T. polyphylla* and tiarellic acid on cysteinyl lukotriene release was measured in BMMB using by $LTC_4$-monoclonal antibody ELISA method.

The result demonstrated that the $IC_{50}$ of tiarellic acid was significant but lower than zileuton known as a 5-lipoxygenase inhibitor( *See* Table 2)

Table 2

| sample | $LTC_4$ release inhibition($IC_{50}$) |
|---|---|
| Extract of *T. polyphylla* | 19.5μg/ml |
| Tiarellic acid | 2.49μM |
| zileuton | 0.11μM |

**Experimental Example 5. The effect of the extract of *T. polyphylla* and tiarellic acid on Airway hyper-**

**responsiveness (AHR)**

24 hours after the final aerosol challenge AHR was measured by using a whole-body plethysmography (OCP3000; Allmedicus, Korea)(Hamelmann E et al., Am J Respir Crit Care Med., 156, pp 766-775, 1997). Each mouse was placed in a brometric phlethysmographic chamber and challenged with aerosolized PBS, followed by increasing concentrations of aerosolized methacholine (12.5, 25 and 50 mg/ml) for 3 min. Bronchoconstriction was recorded for additional 5 mins at each concentration. The highest Penh value of each sample was obtained during each methacholine challenge, and expressed as a percentage of a basal Penh value in response to control (PBS) challenge.

As shown in Table 3, the Penh value of the OVA-treated group was significantly higher than that of the PBS control group (P < 0.05) at any concentration of 5-20 mg/ml of methacholine. In the tiarellic acid+OVA-challenged group, the Penh value was significantly reduced compared with that of the OVA-treated group (P < 0.05). A positive control, zileuton developed as an anti-asthmatic drug, showed a decrease of AHR but less than tiarellic acid( *See* Fig. 2)

Table 3

| Effect of extract of *T. polyphylla* and tiarellic acid on airway hyperresponsiveness (AHR) | | | | |
|---|---|---|---|---|
| sample | Penh value | | | |
| | Metacholine(mg/ml) | | | |
| | 0 | 5 | 10 | 20 |
| OVA-control | 0.73±0.20 | 1.23±0.48 | 2.19±0.58 | 2.89±0.73 |
| Extrac of *T. polyphylla* | 0.60±0.06 | 1.79±0.47 | 3.25±0.75 | 2.54±0.57* |
| Tiarellic acid(30mg/ml) | 0.46±0.08 | 0.96±0.45* | 1.54±0.74* | 2.15±0.52* |
| Zileuton(30mg/ml) | 0.55±0.14 | 1.17±0.59 | 1.97±0.83 | 2.49±0.90 |
| * significant difference from OVA treated group, p<0.05 | | | | |

**Experimental Example 6. The effect of tiarellic acid on OVA-specific IgE**

48 hours after the last challenge, the mice were sacrificed with an overdose of pentobarbital (Sigma P3761), and a tracheotomy was performed. After ice-cold PBS (0.5 ml) was instilled into the lungs, bronchoalveolar lavage fluid (BALF) was obtained by aspiration three times (total 1.5 ml) via tracheal cannulation. BALF was centrifuged and supernatants were collected and stored at -70°C before use. The amount of IL-4, IL-5 and IL-13 in BALF was measured by a specific mouse ELISA kit (R&D Systems; Minneapolis, MN) according to the manufacturer's instructions. The detection limit of the assays was 250 pg/ml.

Plasma was obtained by cardiac puncture after the tracheotomy. Complementary capture and detection antibody pairs for mouse IgE antibodies were purchased from BD OptEIA (San Diego, CA), and the IgE enzyme-linked immunosorbent assay (ELISA) was performed according to the manufacturer's directions. Duplicate samples in plasma were diluted to 1:100. IgE levels in each sample were measured the optical density readings at 450 nm, and OVA-specific IgE concentrations were calculated from a standard curve, which was generated in case recombinant IgE (5-2,000 ng/ml) was used.

As shown in Table 4, the IgE level of tiarellic acid-treated mice was significantly reduced, and zileuton showed similar suppressive effect to tiarellic acid.

Table 4

| sample | OVA-specific IgE |
|---|---|
| OVA-control | 26.0±10.9 |
| OVA+tiarellic acid (30mg/ml)(% inhibition) | 13.3±5.27*(48.8±20.3%) |
| OVA+zileuton (30mg/ml)(% inhibition) | 14.1±1.0*(45.8±15.3%) |
| *significant difference from OVA treated group, p<0.05 | |

**Experimental Example 7. The effect of the extract of *T. polyphylla* and tiarellic acid on cytokine levels**

To determine the effect of extract of *T. polyphylla* and tiarellic acid on the cytokine release in the OVA-induced

asthmatic mice, 48 hours after the last challenge, the levels of cytokines (IL-4, IL-5 and IL-13) in BALF were measured by using ELISA method.

As shown in Table 5, the cytokines in the tiarellic acid-treated group were significantly suppressed; 90.5±4.0%, 54.6±23.0% and 43.7±28.2% more decrease in IL-4, IL-5 and in IL-13 ($P < 0.05$), respectively than a OVA-challenged group. Zileuton also showed more reduced activity than the control but far less than tiarellic acid. These results demonstrate that tiarellic acid significantly reduced the concentration of IL-4, IL-5 and IL-13 in the BALF of the asthmatic model.

Table 5

| sample | IL-4(pg/ml) | IL-5(pg/ml) | IL-13(pg/ml) |
|---|---|---|---|
| OVA-control | 356.1±14.7 | 180.4±17.3 | 145.0±10.9 |
| OVA+extract of *T. polyphylla*(30 mg/ ml)(% inhibition) | 294.7±38.2*(18.9%) | 379.5±94.9*(26.8%) | 32.3±9.7**(62.4%) |
| OVA+tiarellic acid (30 mg/ml)(% inhibition) | 33.8±14.3*(90.5%) | 81.9±41.4*(54.6%) | 81.7±40.9*(43.7%) |
| OVA+zileuton(30 mg/ml)(% inhibition) | 289.5±59.0(18.7%) | 157.8±41.3(12.5%) | 130.1±16.8(10.3%) |
| * significant difference from OVA-control group, p<0.01** significant difference from OVA-control group, p<0.05 | | | |

**Experimental Example 8. Effect of the extract of *T. polyphylla* and tiarellic acid on OVA-induced inflammation in lung tissue**

The lung tissue was fixed for 24 hours on 10% neutral-buffered formalin. After being embedded in paraffin, the tissues were sliced made into 4-μm thickness sections, and stained with H&E solution (hematoxylin; Sigma MHS-16 and eosin, Sigma HT110-1-32). Subsequently, the stained tissue was mounted and cover-slipped with Dako-mounting medium (Dakocytomation; Denmark Carpinteria CA). The degree of cell infiltration in the airway was scored in a double-blind test performed by two independent investigators (Myou S et al., J. Exp. Med., 198, pp1573-1582, 2003). The peri-bronchiole and peri-vascular inflammation was evaluated by specific standard,i.e., scoring of 0-3, 0, no cells; 1, a few cells; 2, a ring of cells 1 to 5 cell-layer deep; 3, a thick ring of cells more than 5 cell-layer deep. To evaluate the suppressive effect of *T. polyphylla* extract and tiarellic acid on the leukocite infiltration, the degree of inflammation was scored by quantitative analysis in lung tissues 48 hours after the last challenge ( *See* Fig. 2).

As shown in Fig 1, tiarellic acid showed most potent suppressive effect on the inflammation in the lung tissues of OVA-induced mice and followed by extract of *T. polyphylla* and zileuton. In the H&E staining of lung tissues leukocytes in the OVA-treated mice were greatly infiltrated into the peri-bronchiole and peri-vascular connective tissue from normal mice. In the extract of *T. polyphylla* or tiarellic acid treated mice, the infiltration of eosinophil-rich leukocytes was significantly attenuated compared with the OVA-treated mice.

**Experimental Example 9. Effect of the extract of *T. polyphylla* and tiarellic acid on carageenan-induced rat-paw edema**

The inhibiting activity of the extract of *T. polyphylla* and tiarellic acid prepared from the above Examples on the edema formation in ICR rat was determined as follows.

The mice were divided into three groups consisting of 6 rats for each group, i.e., T1 treated with only solvent as a negative control group, T2 treated with 50mg/kg of the extract of *T. polyphylla,* T3 treated with 50mg/kg of aspirin respectively. 1 hour after the treatment, 1 % carragenan solution was injected into inner rat ankle to induce edema and the thickness of ankle was measured by Vernier's caliper to determine the degree of the edema. The determined thickness was calculated by following Math Fig.1.

**MathFigure 1**

The thickness ratio of ankle (%)= [(maximum thickness of rat paw edema)-(the thickness of pre-treated rat paw edema)/(the thickness of pre-treated rat paw edema)] x 100

As shown in Table 6, the edema reached to maximum 4 hours after the treatment. Accordingly, it was confirmed that the extract of *T. polyphylla* and tiarellic acid showed potent inhibition effect on rat paw edema.

Table 6

| hr | T1 | T2 | | T3 | |
|---|---|---|---|---|---|
| | The thickness ratio of ankle(%) | The thickness ratio of ankle(%) | Inhibition(%) | The thickness ratio of ankle(%) | Inhibition(%) |
| 0 | 100 | - | - | - | - |
| 1 | 119.2±15.6 | 125.1±1.5 | -5.0±1.3 | 139.3±40.6 | -16.8±34.0 |
| 2 | 158.0±9.0 | 146.4±19.4 | 7.3±12.3 | 170.4±34.5 | -7.8±21.8 |
| 3 | 194.9±12.4 | 166.8±18.8 | 14.4±9.7 | 180.6±28.6 | 7.3±14.7 |
| 4 | 205.9±19.0 | 180.6±26.9 | 12.3±13.1 | 196.9±15.4 | 4.3±7.5 |
| 5 | 201.6±5.1 | 193.4±22.4 | 4.1±11.1 | 198.5±12.6 | 1.5±6.3 |
| T1: controlT2: 50mg/kg of the extract of *T. polyphylla*T3: 50mg/kg of aspirin | | | | | |

Hereinafter, the formulating methods and kinds of excipients will be described, but the present invention is not limited to them. The representative preparation examples were described as follows.

Preparation of injection

Dried powder of Example 1 or tiarellic acid 100mg

Sodium metabisulfite 3.0mg

Methyl paraben 0.8mg

Propyl paraben 0.1mg

Distilled water for injection optimum amount

Injection preparation was prepared by dissolving active component, controlling pH to about 7.5 and then filling all the components in 20 ample and sterilizing by conventional injection preparation method.

Preparation of powder

Dried powder of Example 1 or tiarellic acid 500mg

Corn Starch 100mg

Lactose 100mg

Talc 10mg

Powder preparation was prepared by mixing above components and filling sealed package.

Preparation of tablet

Dried powder of Example 1 or tiarellic acid 200mg

Corn Starch 100mg

Lactose 100mg

Magnesium stearate optimum amount

Tablet preparation was prepared by mixing above components and entabletting.

Preparation of capsule

Dried powder of Example 1 or tiarellic acid 100mg

Lactose 50mg

Corn starch 50mg

Talc 2mg

Magnesium stearate optimum amount

Tablet preparation was prepared by mixing above components and filling gelatin capsule by conventional gelatin preparation method.

Preparation of liquid

Dried powder of Example 1 or tiarellic acid 1000mg

Sugar 20g

Polysaccharide 20g

Lemon flavor 20g

Liquid preparation was prepared by dissolving active component, and then filling all the components in 1000□ ample and sterilizing by conventional liquid preparation method.

<u>Preparation of health food</u>

Dried powder of Example 1 or tiarellic acid 1000mg

Vitamin mixture optimum amount

Vitamin A acetate 70mg

Vitamin E 1.0mg

Vitamin B 0.13mg

Vitamin B 0.15mg

Vitamin $B_6$ 0.5mg

Vitamin $B_{12}$ 0.2mg

Vitamin C 10mg

Biotin 10mg

Amide nicotinic acid 1.7mg

Folic acid 50mg

Calcium pantothenic acid 0.5mg

Mineral mixture optimum amount

Ferrous sulfate 1.75mg

Zinc oxide 0.82mg

Magnesium carbonate 25.3mg

Monopotassium phosphate 15mg

Dicalcium phosphate 55mg

Potassium citrate 90mg

Calcium carbonate 100mg

Magnesium chloride 24.8mg

<u>Preparation of health beverage</u>

Dried powder of Example 1 or tiarellic acid 1000mg

Citric acid 1000mg

Oligosaccharide 100g

Apricot concentration 2g

Taurine 1g

Distilled water 900□

Health beverage preparation was prepared by dissolving active component, mixing, stirred at 85°C for 1 hour, filtered and then filling all the components in 10000 ample and sterilizing by conventional health beverage preparation method.

## Industrial Applicability

As described in the present invention, the extractof *T. polyphylla* and tiarellic acid isolated therefrom show the inhibitory effect of tiarellic acid against $LTC_4$ release in vitro and the suppressive effect on the IgE level and the cytokine (IL-4, IL-5 and IL-13) production, airway hyperresponsiveness, and leukocyte infiltration in OVA-induced asthmatic mince. Therefore, it can be used as the therapeutics or functional health food for use in treating and preventing inflammatory, allergic and asthmatic disease.

## Claims

1. A composition of a butanol soluble fraction of a *Tiarella polyphylla* methanol extract for use in the treatment of asthmatic disease.

2. A functional health food comprising a butanol soluble fraction of a *Tiarella polyphylla* methanol extract together with a sitologically acceptable additive, for use in the prevention and improvement of asthmatic disease.

3. The functional health food for use according to claim 2, wherein said health food is provided as a pill, powder, granule, tablet, chewing tablet, capsule or beverage type.

## Patentansprüche

1. Zusammensetzung einer butanollöslichen Fraktion eines *Tiarella polyphylla* Methanolextrakts für die Verwendung

in der Behandlung von asthmatischen Krankheiten.

2. Funktionelle Reformkost ("functional health food"), umfassend eine butanollösliche Fraktion eines *Tiarella polyphylla* Methanolextrakts zusammen mit einem sitologisch akzeptablem Additiv, für die Verwendung in der Prävention und Verbesserung von asthmatischen Krankheiten.

3. Funktionelle Reformkost für die Verwendung gemäß Anspruch 2, wobei die Reformkost als Pille, Pulver, Granulat, Tablette, Kautablette, Kapsel oder Getränk zur Verfügung gestellt wird.

**Revendications**

1. Composition comprenant une fraction, soluble dans le butanol, d'un extrait au méthanol de *Tiarella polyphylla,* à utiliser dans le traitement de l'asthme.

2. Aliment diététique fonctionnel, comprenant une fraction soluble dans le butanol d'un extrait au méthanol de *Tiarella polyphylla* ainsi qu'un additif sitologiquement adapté, à utiliser pour prévenir et pour diminuer l'asthme.

3. Aliment diététique fonctionnel selon la revendication 2, dans lequel ledit aliment diététique est présenté sous forme de cachet, de poudre, de granule, de comprimé, de comprimé à mâcher, de capsule ou de boisson.

[Fig. 1]

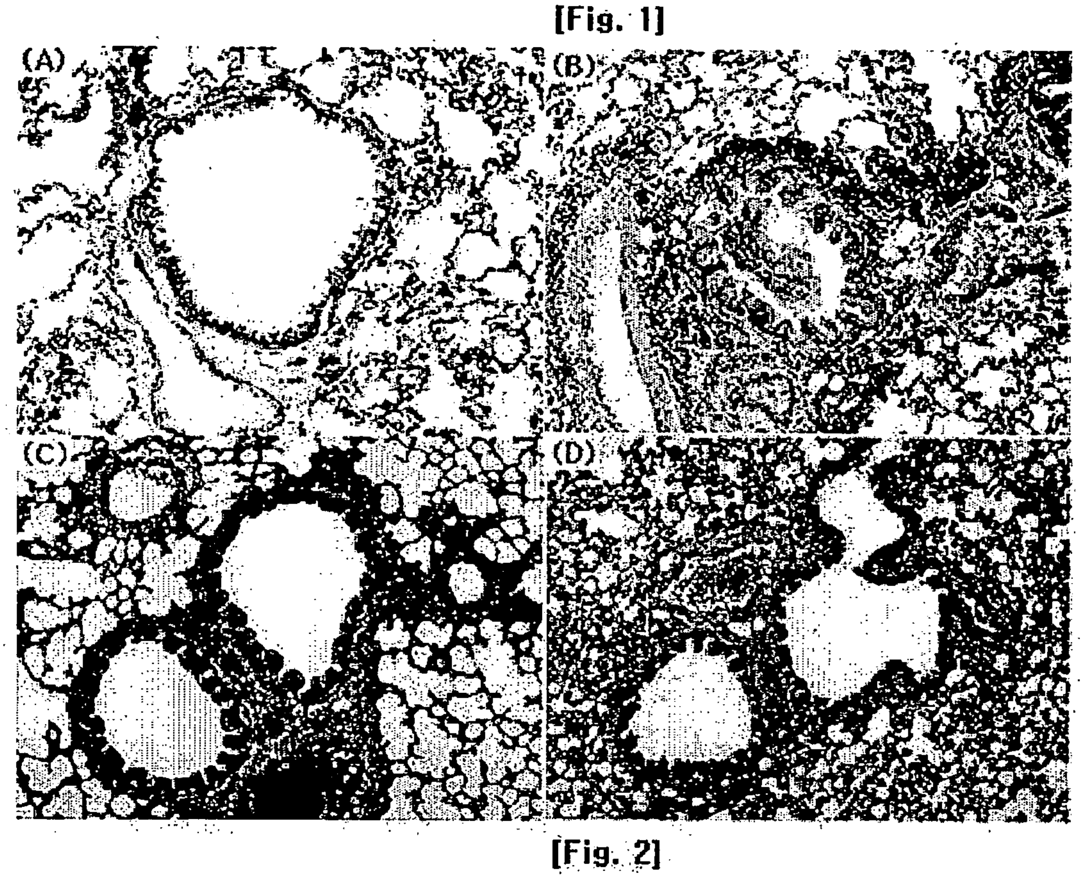
(A)
(B)
(C)
(D)

[Fig. 2]

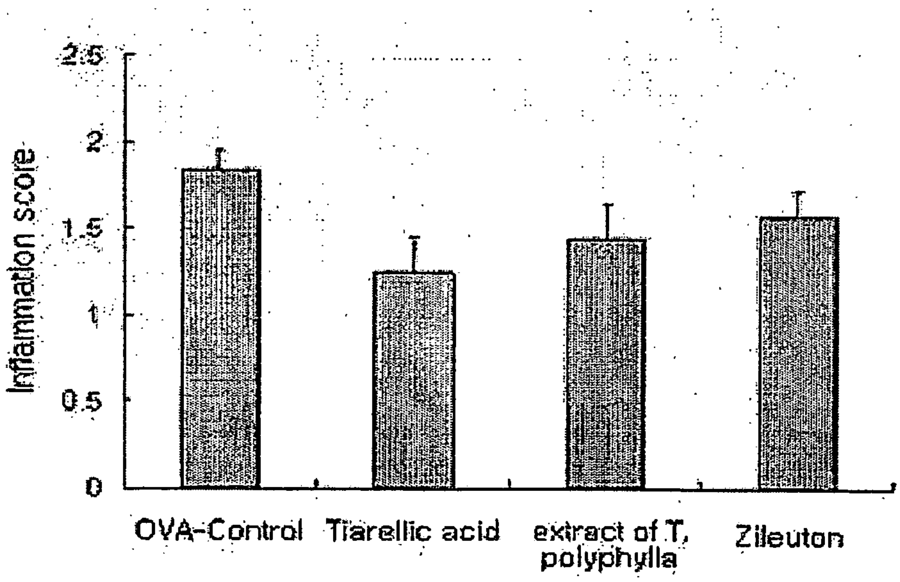
2.5
2
1.5
1
0.5
0
Inflammation score
OVA-Control
Tiarellic acid
extract of T. polyphylla
Zileuton

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Kumar R. K.** *Pharmacol. Ther.,* 2001, vol. 91, 93-104
- **Elias JA et al.** *J Clin Invest.,* 2003, vol. 111, 291-7
- **Moffatt JD.** *Pharmacol Ther,* 2005, vol. 107, 343-57
- **Spina D et al.** *Trends Pharmacol Sci,* 2002, vol. 23, 311-5
- **Bochner B.S. et al.** *Annu. Rev. Immunol.,* 1994, vol. 12, 295-335
- **Bousquet J et al.** *N. Engl. J. Med.,* 1990, vol. 323, 1033-9
- **Riffo-Vasquez Y et al.** *Pharmacol. Ther.,* 2002, vol. 94, 185-211
- **Moffatt J.D.** *Pharmacol. Ther.,* 2005, vol. 107, 343-57
- **Baatjes A.J. et al.** *Pharmacol, Ther.,* 2002, vol. 95, 63-72
- **Park et al.** *Arch Pharm Res.,* 2002, vol. 25, 57-60
- **Moon et al.** *J Ethnopharmacol.,* 2005, vol. 98, 185-189
- **Park et al.** *Arch Pharm Res,* 2002, vol. 25, 57-60
- **Wang Z et al.** *Biol., Pharm. Bull.,* 2001, vol. 24, 159-162
- **Murakami M et al.** *J. Bio. Chem.,* 1995, vol. 39, 22653-22656
- **Lee SH et al.** *Biol. Pharm. Bull.,* 2004, vol. 27, 786-788
- **Hamelmann E et al.** *Am J Respir Crit Care Med.,* 1997, vol. 156, 766-775
- **Myou S et al.** *J. Exp. Med.,* 2003, vol. 198, 1573-1582